# FASCICULE DE BREVET EUROPEEN

(11) **EP 0 912 743 B1**
(45) Date de publication et mention de la délivrance du brevet: **28.09.2005**
(21) Numéro de dépôt: 97933745.8
(22) Date de dépôt: 15.07.1997
(51) Int. Cl.: C12N 15/38, C12N 15/44, C12N 15/40, C12N 15/35, C12N 15/31, A61K 39/295

(54) **FORMULE DE VACCIN POLYNUCLEOTIDIQUE CONTRE LES PATHOLOGIES RESPIRATOIRES ET DE REPRODUCTION DES PORCS**
ZUSAMMENSETZUNG EINES POLYNUKLEOTIDVAKZINS ZUR BEHANDLUNG VON ATEMWEGS- UND FORTPFLANZUNGSBEZOGENEN ERKRANKUNGEN DES SCHWEINS
POLYNUCLEOTIDE VACCINE FORMULA FOR TREATING PORCINE RESPIRATORY AND REPRODUCTIVE DISEASES

(30) Priorité: 19.07.1996 FR 9609338
(43) Date de publication de la demande: 06.05.1999
(73) Titulaire: MERIAL, 69002 Lyon (FR)
(72) Inventeur: AUDONNET, Jean-Christophe, F-69006 Lyon (FR); BOUCHARDON, Annabelle, F-69007 Lyon (FR); BAUDU, Philippe, F-69290 Craponne (FR); RIVIERE, Michel, F-69130 Ecully (FR)
(74) Mandataire: Nargolwalla, Cyra
(86) Numéro de dépôt international: PCT/FR1997/001313
(87) Numéro de publication internationale: WO 1998/003658

(56) Documents cités:
- WO-A-91/00352
- WO-A-95/20660
- WO-A-96/06619
- WO-A-97/23502
- HAYNES J.R. ET AL: 'Particle-mediated nucleic acid immunization' JOURNAL OF BIOTECHNOLOGY vol. 44, no. 1-3, 26 Janvier 1996, pages 37 - 42
- ANDREW M.E. ET AL: 'Protection of pigs against classical swine fever with DNA-delivered gp55' VACCINE vol. 8, 2000, pages 1932 - 1938
- SCHULTZ J. ET AL: 'Update on antiviral DNA vaccine research (1998-2000)' INTERVIROLOGY vol. 43, 2000, pages 197 - 211
- Study of the delivery of the GD gene of PRV to one-day-old piglets by adenovirus or plasmid DNA as ways to by-pass tje inhibition of immune response by colostral antibodies
- LE POTIER M.F. ET AL: 'Study of the delivery of the GD gene of PRV to one-day-old piglets by adenovirus or plasmid DNA as ways to by-pass tje inhibition of immune response by colostral antibodies' SECOND INTERNATIONAL SYMPOSIUM ON THE ERADICATION OF AUJESZKY'S DISEASE VIRUS 06 Août 1995, COPENHAGEN, DENMARK,

## Description

La présente invention est relative à un vaccin permettant la vaccination des porcs contre le virus de la peste porcine classique (HCV). Elle est également relative à une méthode de vaccination corrèspondante.

Au cours des dernières décennies, les méthodes de production porcines ont fondamentalement changé. L'élevage intensif en espace clos s'est généralisé avec comme corollaire le développement dramatique des pathologies respiratoires.

L'ensemble des symptomes de pathologie respiratoire porcine est en général regroupé sous l'appelation complexe de maladie respiratoire des porcs et implique une grande variété d'agents pathogènes, comprenant aussi bien des virus que des bactéries et des mycoplasmes.

Les principaux agents intervenant dans les troubles respiratoires sont Actinobacillus pleuropneumoniae, le virus de l'infertilité et du syndrôme respiratoire (PRRS) encore appelé virus de la maladie mystérieuse, le virus de la maladie d'Aujeszky (PRV) et le virus de la grippe porcine.

D'autres virus entraînent des troubles de la reproduction se traduisant par des avortements, des momifications de foetus et de l'infertilité. Les principaux virus sont PRRS, le parvovirus et le virus de la peste porcine classique (HCV). Secondairement ,les virus PRV grippe porcine et A. pleuropneumoniae peuvent aussi entraîner de tels troubles. Des mortalités peuvent intervenir avec A. pleuropneumoniae, HCV et PRV.

En outre, les interactions entre les microorganismes sont très importants dans le complexe respiratoire porcin. En effet, la plupart des pathogènes bactériens sont des hôtes habituels des zones nasopharyngées et des amygdales chez le jeune animal. Ces pathogènes, qui proviennent de la truie, sont souvent inhalés par les jeunes porcs durant leurs premières heures de vie, avant que l'immunité colostrale soit devenue efficace. Les organismes résidant dans le tractus respiratoire supérieur peuvent envahir le tractus inférieur lorsque les mécanismes de défense respiratoires de l'hôte sont mis à mal par un agent précurseur tel que Actinobacillus pleuropneumoniae ou par des virus. L'invasion pulmonaire peut être très rapide en particulier dans le cas de pathogènes précurseurs tels que Actinobacillus pleuropneumoniae qui produisent des cytotoxines puissantes capables d'endommager les cils des cellules épithéliales respiratoires et les macrophages alvéolaires.

Des infections virales importantes, telles que influenza, infections à coronavirus respiratoires et virus d'Aujeszky, peuvent jouer un rôle dans la pathogénie du complexe respiratoire, au côté de bactéries à tropisme respiratoire et de mycoplasmes.

Enfin certains agents ont une incidence à la fois en respiratoire et en reproduction. Des interactions peuvent aussi se produire sur le plan de la pathologie de la reproduction.

Il paraît donc nécessaire de tenter de mettre au point une prévention efficace contre les principaux agents pathogènes intervenant dans les pathologies respiratoires et de reproduction des porcs.

Les associations développées jusqu'à présent étaient réalisées à partir de vaccins inactivés ou de vaccins vivants et éventuellement de mélanges de tels vaccins. Leur mise en oeuvre pose des problèmes de compatibilité entre valences et de stabilité. Il faut en effet assurer à la fois la compatibilité entre les différentes valences de vaccin, que ce soit au plan des différents antigènes utilisés au plan des formulations elles-mêmes, notamment dans le cas où l'on combine à la fois des vaccins inactivés et des vaccins vivants. Il se pose également le problème de la conservation de tels vaccins combiné et aussi de leur innocuité notamment en présence d'adjuvant. Ces vaccins sont en général assez coûteux.

Les demandes de brevet WO-A-90 11092, WO-A-93 19183, WO-A-94 21797 et WO-A-95 20660 ont fait usage de la technique récemment développée des vaccins polynucléotidiques. On sait que ces vaccins utilisent un plasmide apte à exprimer dans les cellules de l'hôte l'antigène inséré dans le plasmide. Toutes les voies d'administration ont été proposées (intrapéritonéal, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al,. Nature 356, 152-154, 1992) et les injecteurs par jet liquids permettant de transfecter à la fois dans la peau, le muscle, les tissus graisseux et les tissus mammaires (Furth et al., Analytical Biochemistry, 205, 365-368, 1992).

Les vaccins polynucléotidiques peuvent utiliser aussi bien des ADN nus que des ADN formulés par exemple au sein de liposomes de lipides cationiques.

M-F Le Potier et al., (Second International Symposium on the Eradication of Aujeszky's Disease (pseudo rabies) Virus August 6th to 8th 1995 Copenhagen, Denmark) et M. Monteil et al. (Les Journées d'Animation Scientifique du Département de Pathologie Animale, INRA-ENV, Ecole Nationale Vétérinaire de LYON, 13-14 déc 1994) ont tenté de vacciner les porcs contre le virus de la maladie d'Aujeszky à l'aide d'un plasmide permettant l'expression du gène gD sous le contrôle d'un promoteur fort, le promoteur majeur tardif de l'adénovirus de type 2. Malgré une réponse en anticorps de bon niveau, aucune protection n'a pu être mise en évidence. Or, des résultats satisfaisants en matière de protection ont été enregistrés après inoculation aux porcs d'un adénovirus recombinant dans lequel a été inséré le gène gD et le même promoteur, prouvant que la glycoprotéine gD Serait suffisante pour induire une protection chez le porc.

L'art antérieur ne donne aucun résultat de protection chez le porc par la méthode de la vaccination polynucléotidique.

L'invention se propose de fournir une formule de vaccin permettant d'assurer une vaccination des porcs contre la peste porcine classique.

Un autre objectif de l'invention est de fournir une telle formule de vaccin qui soit de mise en oeuvre aisée et peu coûteuse.

Un autre objectif encore de l'invention est de fournir une telle formule de vaccin et une méthode de vaccination des porcs qui permette d'obtenir une protection, avec un niveau élevé d'efficacité et de longue durée, ainsi qu'une bonne innocuité et une absence de résidus.

La présente invention a donc pour objet un vaccin porcin comprenant un plasmide contenant le gène E2 du virus de la peste porcine classique (HCV), ce vaccin induisant une protection des porcs contre la peste porcine calssique.

La présente invention a également pour objet un vaccin comprenant le gène E1 et le gène E2 du virus de la peste porcine classique (HCV), les deux gènes pouvant être combinés dans un seul et unique plasmide ou dans deux plasmides différents.

Par gène d'agent pathogène, on entend aon seulement le gène complet, mais aussi les séquences nucléotidiques différentes, y compris fragments, conservant la capacité à induire une réponse protectrice. La notion de gène recouvre les séquences nucléotidiques équivalentes à celles décrites précisément dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

On peut utiliser un gène E2 ou également des gènes E1 et E2 combinés, dans deux plasmides différents ou éventuellement dans un seul et même plasmide.

Le vaccin selon l'invention pourra se présenter sous un volume de dose compris de manière générale entre 0,1 et 10 ml, et en particulier entre 1 et 5 ml notamment pour les vaccinations par voie intramusculaire.

La dose sera générallement comprise entre 10 ng et 1 mg, de préférence entre 100 ng et 500 µg et préférentiellement entre 1 µg et 250 µg par type de plasmide.

On utilisera de préférence des plasmides nus simplement placés dans le véhicule de vaccination qui sera en général de l'eau physiologique (Nacl 0,9 %), de l'eau ultrapure, du tampon TE, etc. On peut bien entendu utiliser toutes les formes de vaccin polynucléotidiques décrites dans l'art antérieur.

Chaque plasmide comprend un promoteur apte à assurer l'expression du gène inséré sous sa dépendance dans les cellules hôtes. Il s'agira en général d'un promoteur eucaryote fort et en particulier d'un promoteur précoce du cytomégalovirus CMV-IE, d'origine humaine ou murine, ou encore éventuellement d'une autre origine telle que rat, cochon, cobaye.

De manière plus générale, le promoteur pourra être soit d'origine virale, soit d'origine cellulaire. Comme promoteur viral, on peut citer le promoteur précoce ou tardif du virus SV40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, par exemple le promoteur de la desmine (Bolmont et al., Journal of Submicroscopic Cytology and Pathology, 1990, 22, 117-122 ; et ZHENLIN et al., Gene, 1989, 78, 243-254), ou encore le promoteur de l'actine.

Lorsque plusieurs gènes sont présents dans le même plasmide, ceux-ci peuvent être présentés dans la même unité de transcription ou dans deux unités différentes.

Le vaccin monovalent peut être utilisé (i) pour la préparation d'une formule de vaccin polyvalent, (ii) à titre individuel contre la pathologie propre, (iii) associées à un vaccin d'un autre type (entier vivant ou inactivé, recombinant, sous-unité) contre une autre pathologie, ou (iv) comme rappel d'un vaccin comme décrit ci-après.

La présente invention a en effet encore pour objet l'utilisation d'un ou de plusieurs plasmides selon l'invention pour la fabrication d'un vaccin destiné à vacciner les pores primo-vaccinés au moyen d'un premier vaccin classique du type de ceux de la technique antérieure, à savoir notamment choisi dans la groupe consistant en vaccin entier vivant, vaccin entier inactivé, vaccin de sous-unité, vaccin recombinant, ce premier vaccin (monovalent) présentant (c'est-à-dire contenant ou pouvant exprimer) le ou les antigène(s) codé(s) par le ou les plasmides ou antigène(s) assurant une protection croisée. De manière remarquable, le vaccin polynucléotidique a un effet de rappel puissant se traduisant par une amplification de la réponse immunitaire et l'installation d'une immunité de longue durée.

De manière générale, les vaccins de primo-vaccination pourront être choisist parmi les vaccins commerciaux disponibles auprès des différents productours de vaccins vétérinaires.

L'invention a aussi pour objet un kit de vaccination regroupant un vaccin de primo-vaccination tel que décrit ci-dessus et une formule de vaccin selon l'invention pour le rappel. Elle a aussi trait à une formule de vaccin selon l'invention accompagnée d'une notice indiquant l'usage de cette formule comme rappel d'une primo-vaccination telle que décrite ci-avant.

La présente invention a également pour objet l'utilisation des plasmides décrits plus haut, pour la réalisation d'un vaccin destiné à vacciner le porc contre la peste porcine classique. La vaccination des porcs contre HCV comprend l'administration d'une dose efficace d'un vaccin tel que décrit plus haut. Cette méthode de vaccination comprend l'administration d'une ou de plusieurs doses de vaccin, ces doses pouvant être administrées successivement dans un court laps dé temps et/ou successivement à des moments éloignés l'un de l'autre.

Les vaccins selon l'invention pourront être administrés dans le cadre de cette méthode de vaccination, par les différentes voies d'administration proposées dans l'art antérieur pour la vaccination polynucléotidique et au moyen des techniques d'administration connues. On pourra notamment utiliser la vaccination par voie intradermique à l'aide d'un injecteur par jet liquide, de préférence par jets multiples, et en particulier un injecteur utilisant une tête d'injection munie de plusieurs trous ou buses, notamment comprenant de 5 à 6 trous ou buses, tel que l'appareil Pigjet fabriqué et distribué par la société Endoscoptic, Laons, France.

Le volume de dose pour un tel appareil sera réduit de préférence entre 0,1 et 0,9 ml, en particulier entre 0,2 et 0,6 ml et avantageusement entre 0,4 et 0,5 ml, le volume pouvant être appliqué en une ou plusieurs, de préférence 2, applications.

L'invention a encore pour objet la méthode de vaccination consistant à faire une primo-vaccination telle que décrite ci-dessus et un rappel avec une formule de vaccin selon l'invention. Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, après un délai de préférence de 2 à 6 semaines, on assure l'administration du vaccin polyvalent ou monovalent selon l'invention.

L'invention concerne aussi la méthode de préparation des formules de vaccin, à savoir la préparation des valences et leurs mélanges, telle qu'elle ressort de cette description.

L'invention va être maintenant décrite plus en détails à l'aide de modes de réalisation de l'invention pris en référence aux dessins annexés.

### Liste des figures

Figure N° 1 : Plasmide pVR1012
Figure N° 2 : Plasmide pAB069

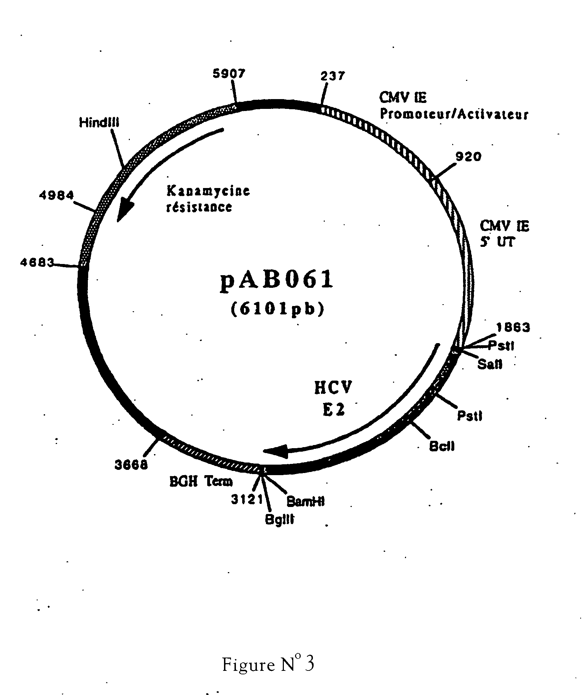

Figure N° 3 : Plasmide pAB061

### Liste des séquences SEQ ID N°

SEQ ID N° 1 : Oligonucléotide AB126
SEQ ID N° 2 : Oligonucléotide AB127
SEQ ID N° 3 : Oligonucléotide AB118
SEQ ID N° 4 : Oligonucléotide AB119

### EXEMPLES

### Exemple 1 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 2 : Extraction des ADNs génomiques viraux:

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM; pH 8,0). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 3 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénol-chloroforme" décrite par P. Chomczynski et N. Sacchi (Anal. Biochem. 1987. 162. 156-159).

### Exemple 4 : Techniques de biologie moléculaire

Toutes les constructions de plasmides ont été réalisées en utilisant les techniques standards de biologie moléculaire décrites par J. Sambrook *et al*. (*Molecular Cloning: A Laboratory Manual*. 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).

### Exemple 5 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (J. Sambrook *et al*. (*Molecular Cloning: A Laboratory Manual*. 2^{nd} Edition. Cold Spring Harbor Laboratory. Cold Spring Harbor. New York. 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcool isoamylique (25:24:1) avant d'être digéré par les enzymes de restriction.

### Exemple 6 : plasmide pVR1012

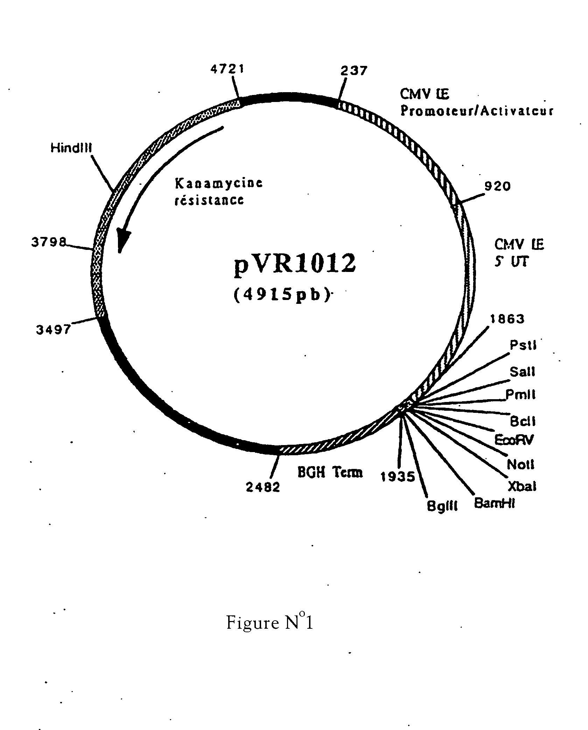

Le plasmide pVR1012 (Figure N° 1) a été obtenu auprès de Vical Inc. San Diego, CA, USA. Sa construction a été décrite dans J. Hartikka *et al*. (Human Gene Therapy. 1996. 7. 1205-1217).

### Exemple 7 : Construction du plasmide pAB069 (gène Peste porcine HCV E1).

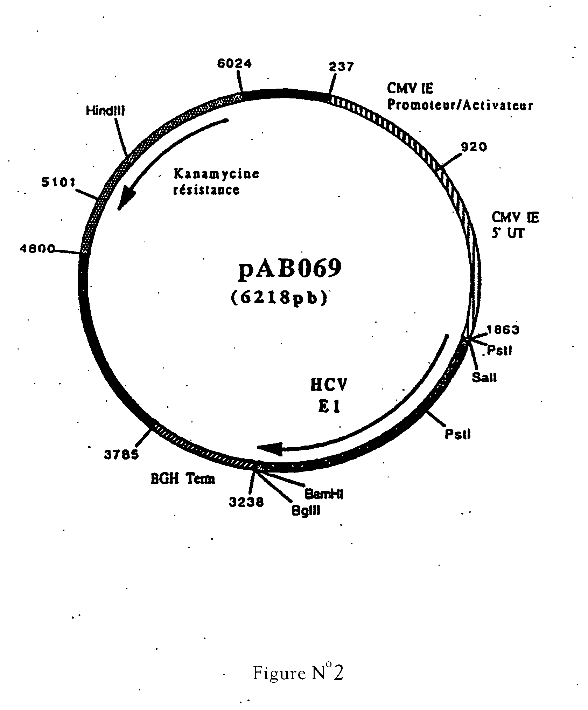

Une réaction RT-PCR selon la technique décrite dans l'exemple 5 a été réalisée avec l'ARN génomique du virus de la peste porcine (Hog Cholera Virus) (HCV) (Souche Alfort) (G. Meyers *et al*., Virology. 1989. 171, 18-27), préparé selon la technique décrite dans l'exemple 4, et avec les oligonucléotides suivants: 
AB126 (36 mer) (SEQ ID N° 1:)
5'ACGCGTCGACATGAAACTAGAAAAAGCCCTGTTGGC 3'
 
AB127 (34 mer) (SEQ ID N° 2)
5'CGCGGATCCTCATAGCCGCCCTTGTGCCCCGGTC 3'
 pour isoler la séquence codant pour la protéine E1 du virus HCV sous la forme d'un fragment RT-PCR de 1363 pb. Après purification, ce fragment a été digéré par *Sal*I et *Bam*HI pour donner un fragment SalI-BamHI de 1349 pb. Ce fragment a été ligaturé avec le vecteur pVR1012 (exemple 6), préalablement digéré avec *Sal*I et *Bam*HI, pour donner le plasmide pAB069 (6218 pb) (Figure N° 2).

### Exemple 8 : Construction du plasmide pA8061 (gène Peste porcine HCV E2).

Une réaction RT-PCR selon la technique décrite dans l'exemple 5 a été réalisée avec l'ARN génomique du virus de la peste porcine (Hog Cholera Virus) (HCV) (Souche Alfort) (G. Meyers *et al*., Virology, 1989, 171, 18-27), préparé selon la technique décrite dans l'exemple 4, et avec les oligonucléotides suivants: 
AB118 (36 mer) (SEQ ID N° 3)
5'ACGCGTCGACATGTCAACTACTGCGTTTCTCATTTG 3'
 
AB119 (33 mer) (SEQ ID N° 4)
5'CGCGGATCCTCACTGTAGACCAGCAGCGAGCTG 3'
 pour isoler la séquence codant pour la protéine E2 du virus HCV sous la forme d'un fragment RT-PCR de 1246 pb. Après purification, ce fragment a été digéré par *Sal*I et *Bam*HI pour donner un fragment Sall-BamHI de 1232 pb. Ce fragment a été ligaturé avec le vecteur pVR1012 (exemple 6), préalablement digéré avec *Sal*I et *Bam*HI, pour donner le plasmide pAB061 (6101 pb) (Figure N° 30).

### Exemple 9 : Préparation et purification des plasmides

Pour la préparation des plasmides destinés à la vaccination des animaux, on peut utiliser toute technique permettant d'obtenir une suspension de plasmides purifiés majoritairement sous forme superenroutée. Ces techniques sont bien connues de l'homme de l'art. On peut citer en particulier la technique de lyse alcaline suivie de deux ultracentrifugations successives sur gradient de chlorure de césium en présence de bromure d'éthidium telle que décrite dans J. Sambrook *et al*. (*Molecular Cloning: A Laboratory Manual*. 2^{nd} Edition. Cold Spring Harbor Laboratory. Cold Spring Harbor. New York. 1989). On peut se référer également aux demandes de brevet PCT WO 95/21250 et PCT WO 96/02658 qui décrivent des méthodes pour produire à l'échelle industrielle des plasmides utilisables pour la vaccination. Pour les besoins de la fabrication des vaccins , les plasmides purifiés sont resuspendus de manière à obtenir des solutions à haute concentration ( > 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 10 : Fabrication des vaccins associés

Les divers plasmides nécessaires à la fabrication d'un vaccin associé sont mélangés à partir de leurs solutions concentrées (exemple 16). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACl à 0,9 %, soit du tampon PBS.
Des formulations particulières telles que les liposomes, les lipides cationiques, peuvent aussi être mises en oeuvre pour la fabrication des vaccins.

### Exemple 11 : Vaccination des porcs

Les porcs sont vaccinés avec des doses de 100 µg, 250 µg ou 500 µg par plasmide.
Les injections peuvent être réalisées à l'aiguille par voie intramusculaire. Dans ce cas, les doses vaccinales sont administrées sous un volume de 2 ml. Les injections peuvent être réalisées par voie intradermique en utilisant un appareil d'injection à jet liquide (sans aiguille) délivrant une dose de 0,2 ml en 5 points (0,04 ml par point d'injection) (par exemple, appareil "PIGJET"). Dans ce ces, les doses vaccinales sont administrées sous des volumes de 0,2 ou 0,4 ml, ce qui correspond respectivement à une ou à deux administrations. Lorsque deux administrations successives sont pratiquées au moyen de l'appareil PIGJET, ces administrations sont réalisées de manière décalée, de façon que les deux zones d'injection soient séparées l'une de l'autre par une distance d'environ 1. à 2 centimètres.

## Revendications

1. Vaccin porcin comprenant un plasmide contenant le gène E2 du virus de la peste porcine (HCV), ce vaccin induisant une protection des porcs contre la peste porcine classique.

2. Vaccin selon la revendication 1, dans lequel le plasmide comprend en outre le gène E1.

3. Vaccin selon la revendication 1, qui comprend en outre un autre plasmide contenant le gène E1.

4. Vaccin selon l'une quelconque des revendications 1 à 3, dans lequel le plasmide comprend le promoteur précoce du cytomégalovirus CMV-IE.

5. Vaccin selon l'une quelconque des revendications 1 à 3, dans lequel le plasmide comprend un promoteur choisi parmi : promoteur précoce du virus SV40, promoteur tardif du virus SV40, promoteur LTR du virus du Sarcome de Rous, promoteur de la desmine, promoteur de l'actine.

6. Utilisation d'un plasmide selon l'une quelconque des revendications 1 à 5, pour la fabrication d'un vaccin destiné à vacciner des porcs primo-vaccinés au moyen d'un premier vaccin choisi dans le groupe consistant en vaccin entier vivant, vaccin entier inactivé, vaccin de sous-unité, vaccin recombinant, ce premier vaccin présentant l'antigène codé par le vaccin polynucléotidique ou un antigène assurant une protection croisée.

7. Kit de vaccination regroupant un vaccin selon l'une quelconque des revendications 1 à 5, et un vaccin choisi dans le groupe consistant en vaccin entier vivant, vaccin entier inactivé, vaccin de sous-unité, vaccin recombinant, ce premier vaccin présentant l'antigène codé par le vaccin polynucléotidique ou un antigène assurant une protection croisée, pour une administration de ce premier vaccin en primo-vaccination et pour un rappel avec ledit vaccin selon l'une quelconque des revendications 1 à 5.

8. vaccin selon l'une quelconque des revendications 1 à 5, accompagné d'une notice indiquant que cette formule est utilisable en rappel d'un premier vaccin choisi dans le groupe consistant en vaccin entier vivant, vaccin entier inactivé, vaccin de sous-unité, vaccin recombinant, ce premier vaccin présentant l'antigène codé par ledit vaccin selon l'une quelconque des revendications 1 à 5 ou un antigène assurant une protection croisée.

9. Utilisation d'un ou de plasmides tels que décrits dans l'une quelconque des revendications 1 à 5, comprenant le gène E2 de HCV pour la réalisation d'un vaccin destiné à vacciner le porc, contre la peste porcine classique, par voie intramusculaire ou par voie intradermique à l'aide d'un injecteur par jet liquide.

## Patentansprüche

1. Schweineimpfstoff, umfassend ein Plasmid, enthaltend das E2-Gen des Virus der klassischen Schweinepest (HCV), wobei dieser Impfstoff bei den Schweinen einen Schutz gegen die klassische Schweinepest induziert.

2. Impfstoff gemäß Anspruch 1, wobei das Plasmid außerdem das E1-Gen umfasst.

3. Impfstoff gemäß Anspruch 1, der außerdem ein anderes Plasmid umfasst, das das E1-Gen enthält.

4. Impfstoff gemäß einem der Ansprüche 1 bis 3, wobei das Plasmid den frühen Promotor des Cytomegalovirus CMV-IE umfasst.

5. Impfstoff gemäß einem der Ansprüche 1 bis 3, wobei das Plasmid einen Promotor umfasst, der ausgewählt ist aus : früher Promotor des SV40-Virus, später Promotor des SV40-Virus, LTR-Promotor des Rousschen Sarkoms, Desmin-Promotor, Aktin-Promotor.

6. Verwendung eines Plasmids gemäß einem der Ansprüche 1 bis 5, zur Herstellung eines Impfstoffes für die Impfung von primär geimpften Schweinen mit einem Primärimpfstoff, ausgewählt aus der Gruppe bestehend aus einem Lebendvollimpfstoff, einem inaktivierten Vollimpfstoff, einem Impstoff aus einer Untereinheit, einem rekombinanten Impfstoff, wobei der Primärimpfstoff das Antigen präsentiert, das durch den Polynucleotidimpfstoff codiert wird, oder ein Antigen, das einen Kreuzschutz gewährleistet.

7. Impfstoffkit, der einen Impfstoff gemäß einem der Ansprüche 1 bis 5, und einen Impfstoff umfasst, der ausgewählt ist aus der Gruppe bestehend aus einem Lebendvollimpfstoff, einem inaktivierten Vollimpfstoff, einem Impfstoff aus einer Untereinheit, einem rekombinanten Impfstoff, wobei der Primärimpfung und für eine Wiederauffrischung mit dem Impfstoff gemäß einem der Ansprüche 1 bis 5.

8. Impfstoff gemäß einem der Ansprüche 1 bis 5, mit einem Hinweis, dass diese Formel bei einer Wiederauffrischung des Primärimpfstoffes verwendet werden kann, der ausgewählt ist aus der Gruppe bestehend aus einem Lebendvollimpfstoff, einem inaktivierten Vollimpfstoff, einem Impfstoff aus einer Untereinheit, einem rekombinanten Impfstoff, wobei der Primärimpfstoff das Antigen präsentiert, das durch den Impfstoff gemäß einem der Ansprüche 1 bis 5 codiert wird, oder ein Antigen, das einen Kreuzschutz gewährleistet.

9. Verwendung eines Plasmids oder der Plasmide wie in eineme der Ansprüche 1 bis 5 beschrieben, umfassend das E2-Gen des HCV zur Herstellung eines Impfstoffes zur intramuskulären oder intradermalen Impfung von Schweinen gegen die klassische Schweinepest mit Hilfe eines Injektors mit einem Flüssigkeitsstrahl.

## Claims

1. A swine vaccine comprising a plasmid containing the E2 gene of the virus of classical swine fever (HCV), said vaccine inducing protection for swine against classical swine fever.

2. A vaccine according to claim 1 wherein the plasmid further includes the E1 gene.

3. A vaccine according to claim 1 which further comprises another plasmid containing the E1 gene.

4. A vaccine according to any one of claims 1 to 3 wherein the plasmid comprises the early promoter of the cytomegalovirus CMV-IE.

5. A vaccine according to any one of claims 1 to 3 wherein the plasmid comprises a promoter selected from: early promoter of SV40 virus, late promoter of SV40 virus, promoter LTR of the Rous Sarcoma virus, promoter of desmin and promoter of actin.

6. Use of a plasmid according to any one of claims 1 to 5 for the production of a vaccine intended for vaccinating swine which are primovactinated by means of a first vaccine selected from the group consisting of a live whole vaccine, an inactivated whole vaccine, a subunit vaccine and a recombinant vaccine, said first vaccine having the antigen coded by the polynucleotide vaccine or an antigen affording crossed protection.

7. A vaccination kit combining a vaccine according to any one of claims 1 to 5 and a vaccine selected from the group consisting of a live whole vaccine, an inactivated whole vaccine, a subunit vaccine and a recombinant vaccine, said first vaccine having the antigen coded by the polynucleotide vaccine or an antigen affording crossed protection, for an administration of said first vaccine in primovaccination and for a booster with said vaccine according to any one of claims 1 to 5.

8. A vaccine according to any one of claims 1 to 5 accompanied by a notice specifying that said formula can be used as a booster for a first vaccine selected from the group consisting of a live whole vaccine, an inactivated whole vaccine, a subunit vaccine and a recombinant vaccine, said first vaccine having the antigen coded by said vaccine according to any one of claims 1 to 5 or an antigen affording crossed protection.

9. Use of a plasmid or plasmids as set forth in any one of claims 1 to 5 comprising the E2 gene of HCV for the production of a vaccine intended for vaccinating swine against classical swine fever by intramuscular administration or by intradermic administration by means of a liquid-jet injector.
